# EUROPEAN PATENT SPECIFICATION

(11) **EP 1 467 711 B1**
(45) Date of publication and mention of the grant of the patent: **21.03.2007**
(21) Application number: 03700947.9
(22) Date of filing: 23.01.2003
(51) Int. Cl.: A61K 9/16, C12N 15/89

(54) **DNA DOSAGE FORMS**
DARREICHUNGSFORMEN FÜR DNS
FORMES POSOLOGIQUES D'ADN

(30) Priority: 25.01.2002 GB 0201735; 25.01.2002 GB 0201736
(43) Date of publication of application: 20.10.2004
(73) Proprietor: GLAXO GROUP LIMITED, Greenford, Middlesex UB6 0NN (GB)
(72) Inventor: CATCHPOLE, Ian, Richard GlaxoSmithKline, Stevenage, Hertfordshire SG1 2NY (GB)
(74) Representative: Easeman, Richard Lewis
(86) International application number: PCT/GB2003/000336
(87) International publication number: WO 2003/061629

(56) References cited:
- EP-A- 1 138 337
- WO-A-95/19799
- WO-A-97/40839

## Description

The present invention relates to DNA pharmaceutical formulations, and preferably those formulations that are suitable for ballistic delivery into the skin of the human body. The present invention provides a novel DNA pharmaceutical agent dosage form, having a dense core element which is coated with an amorphous solid reservoir medium containing the DNA pharmaceutical agent in solid solution or suspension within it. The dense core element is preferably a small metal bead suitable for ballistic delivery of the agent into a cell, commonly such beads are roughly spherical gold or tungsten microbeads of an average particle size in the range of between 0.5 to 10 micrometers in diameter. Preferably the solid pharmaceutical reservoir medium coating the beads is a polyol, preferably being a polyol in an amorphous state. Preferably the polyol is a carbohydrate such as trehalose or sucrose. The solid pharmaceutical reservoir medium may further comprise a stabilising agent that inhibits the degradative effects of free radicals, such as a free radical scavenger or a metal ion chelator. The DNA pharmaceutical formulations of the present invention are storage stable, in that the DNA is stabilised in its supercoiled form, and only substantially release the DNA after administration to the skin. Furthermore, vaccine delivery devices for the administration of the DNAvaccines into the skin are provided, methods of their manufacture, and their use in medicine.

The skin represents a significant barrier to external agents. A summary of human skin is provided in Dorland's Illustrated Medical Dictionary, 28^{th} Edition. Starting from the external layers, working inwards, the skin comprises the epithelium consisting of the stratum corneum and the viable epithelium, and underlying the epithelium is the dermis. The viable epithelium consists of four layers: Stratum corneum, Stratum lucidium, Stratum granulosum, Stratum spinosum, and Stratum basale. The *epithelium* (including all five layers) is the outermost non-vascular layer of the skin, and varies between 0.07 and 0.12 mm thick (70-120 µm). The epithelium is populated with keratinocytes, a cell that produces keratin and constitutes 95% of the dedicated epidermal cells. The other 5% of cells are melanocytes. The underlying dermis is normally found within a range of 0.3 to about 3 mm beneath the surface of the stratum corneum, and contains sweat glands, hair follicles, nerve endings and blood vessels.

The stratum corneum dominates the skin permeability barrier and consists of a few dozen horny, keratinised epithelium layers. The narrow interstices between the dead or dying keratinocytes in this region are filled with crystalline lipid multilamellae. These efficiently seal the interstices between the skin or body interior and the surroundings by providing a hydrophobic barrier to entry by hydrophilic molecules. The stratum corneum being in the range of 30-70 µm thick.

Langerhans cells are found throughout the basal granular layer of the viable epithelium (stratum spinosum and stratum granulosum, (Small Animal Dermatology - Third Edition, Muller - Kirk - Scott, Ed: Saunders (1983)) and are considered to play an important role in the immune system's initial defence against invading organisms. This layer of the skin therefore represents a suitable target zone for certain types of vaccine.

Conventional modes for administration of pharmaceutical agents into or across the skin, most commonly by hypodermic needle and syringe, are associated with numerous disadvantages. Such disadvantages include pain, the requirement for trained professionals to administer the agent, and also the risk of needle-stick injuries to the administrator with the accompanying risk of infection with a blood born disease. As such, there is a need to improve the method of administration of all types of pharmaceutical into or through the skin.

A number of alternative approaches have been described in order to overcome the problems of administering agent across the stratum corneum, including various devices for the ballistic delivery of vaccines in supersonic gas flow.

DNA vaccines usually consist of a bacterial plasmid vector into which is inserted a strong viral promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example *E.coli* and then isolated and prepared in an appropriate medium, depending upon the intended route of administration, before being administered to the host. Following administration the host cells produce the plasmid encoded protein or peptide. The plasmid vectors are generally made without an origin of replication which is functional in eukaryotic cells, in order to prevent plasmid replication in the mammalian host and integration within chromosomal DNA of the animal concerned. Information in relation to DNA vaccination is provided in Donnelly *et al* "DNA vaccines" *Ann. Rev Immunol.* 1997 15: 617-648, the disclosure of which is included herein in its entirety by way of reference.

Plasmid based delivery of genes, particularly for immunisation or gene therapy purposes is known. For example, administration of naked DNA by injection into mouse muscle is outlined in WO90/11092. Johnston et al WO 91/07487 describe methods of transferring a gene to veterbrate cells, by the use of microbeads onto which a polynucleotide encoding a gene of interest has been precipitated, and accelerating the DNA/microbeads such that they penetrate the target cell. Devices for administration of gold or tungsten beads coated with DNA into cells of the skin are described in US 5,630,796; WO 96/04947; WO 96/12513; WO 96/20022; WO 97/34652; WO 97/48485; WO 99/01168; WO 99/01169. Methods of vaccination using crystalline forms of ballistically delivered pharmaceutical agent are described in WO 99/27961. The present invention provides improved DNA dosage forms for use needleless ballistic delivery devices such as those described in the above publications. The formulations wherein DNA is precipitated onto gold beads as described in the art have the problem that it is difficult to co-formulate the DNA with additional agents/excipients. The present invention provides a method of co-formulation of additional agents.

Solid dosage forms comprising a pharmaceutical agent (including DNA plasmids) and a stabilising polyol (such as a sugar) wherein the dosage forms are in the form of ballistically delivered powders are described in WO 96/03978. The stabilisation of agents in amorphous sugar glasses has been described in US 5,098,893.

Sugars used in pharmaceutical formulations can be either crystalline or amorphous. Amorphous solids are distinguished from crytalline by their lack of three-dimensional long-range order found in crystalline materials. Amorphous solids are similar to liquids at a molecular level wherein the molecules are randomly arranged. Amorphous sugars impart stability to pharmaceutical formulations when stored at temperatures below the glass transition temperature. Amorphous sugars exhibit a property in which there is a change in the mobility of the molecules in the sugar matrix below a temperature called the glass transition temperature. Below this temperature (Tg), amorphous sugars exist in a glassy state and above this temperature in a rubbery state. At temperatures below Tg the mobility of the sugar molecules and any molecules associated or trapped in the sugar matrix is extremely low giving rise to long-term stability of such formulations. In other words, the formation of a glass dramatically reduces the diffusional rates of the molecules. This is also accompanied by a decrease in the heat capacity at constant pressure (Cp) by 40 to 100%. This transition can be readily observed by sensitive thermal techniques like differential scanning calorimetry (Duddu, S.P., Zhang G, and Dal Monte, P. R., 1997., Pharm Res., 14: 596-600). The stabilizing properties of sugars have also been attributed to their hydrogen bonding properties with biological molecules like proteins.

It is desirable for DNA pharmaceutical agents to be delivered in a supercoiled form. Supercoiled DNA in liquid pharmaceutical preparations are known to degrade over time resulting in the loss of the supercoiled structure and associated formation of open circle or linear DNA structures (Evans *et al.,* 2000, Journal of Pharmaceutical Sciences, 89(1), 76-87; WO 97/40839). One mechanism by which this chain scission reaction may occur is oxidation of the DNA by free hydroxyl radicals produced from dissolved oxygen in the DNA solutions, a process that is catalysed by metal ions. The free radical formation reaction may be catalysed by several transition metal ions, the most common of which, however, are iron and copper ions (Fe⁺³, Fe⁺², Cu⁺² or Cu⁺¹; Evans *et al. supra*).

The instability of supercoiled DNA is apparent when the DNA is in liquid solution. However, removal of trace metal ions from supercoiled DNA containing liquid solutions with metal ion chelators, and/or mopping up free radicals in solution by non-reducing free radical scavengers stabilises the DNA in the supercoiled form and protects the DNA from oxidation (WO 97/40839). The problem of stabilisation of dry forms of DNA once coated onto a gold or tungsten bead has hitherto not been addressed in the art. Surprisingly, the present inventors have observed that dry forms of DNA, without the technology of the present invention, when coated onto gold or tungsten microbeads are also unstable.

The present invention overcomes these problems and provides a DNA delivery dose which is capable of administering and releasing the DNA agents efficiently into the skin, with or without additional excipients, and also in which the DNA is stabilised in its supercoiled form.

### DESCRIPTION OF FIGURES

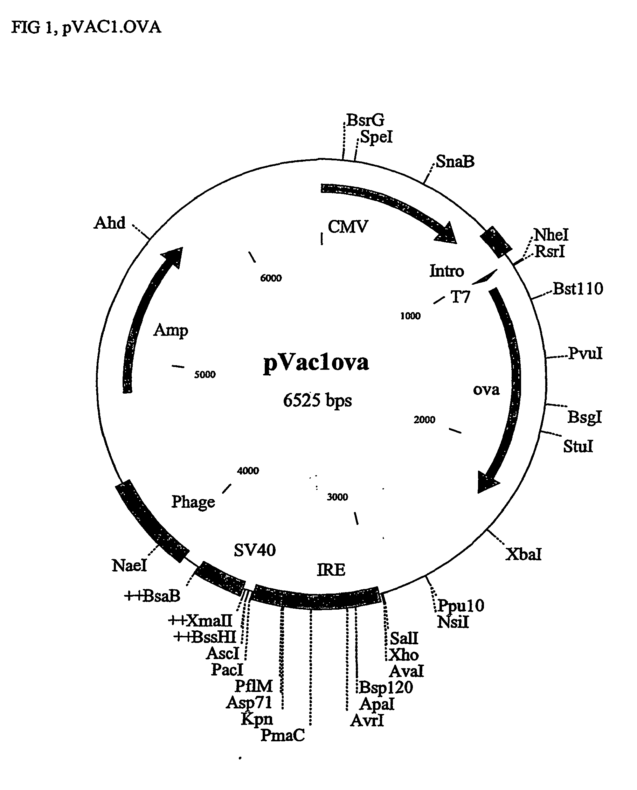

**FIG 1,** pVAC1.ova

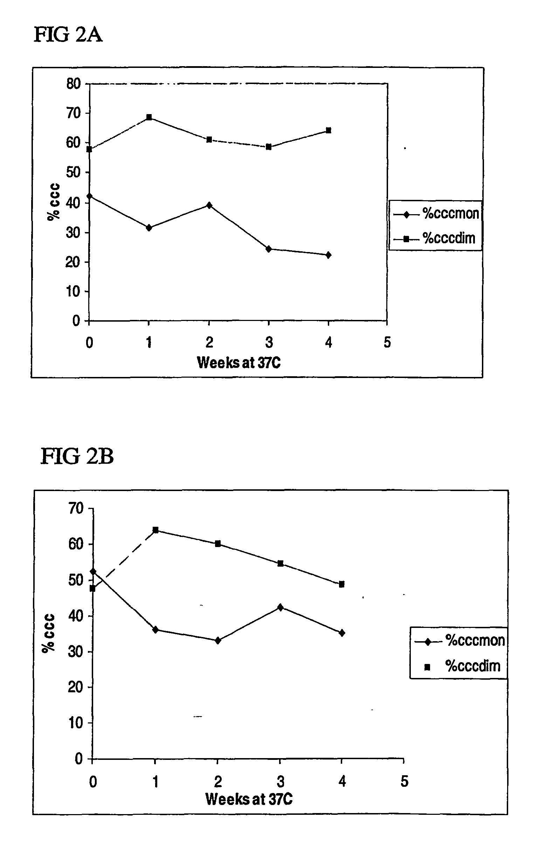

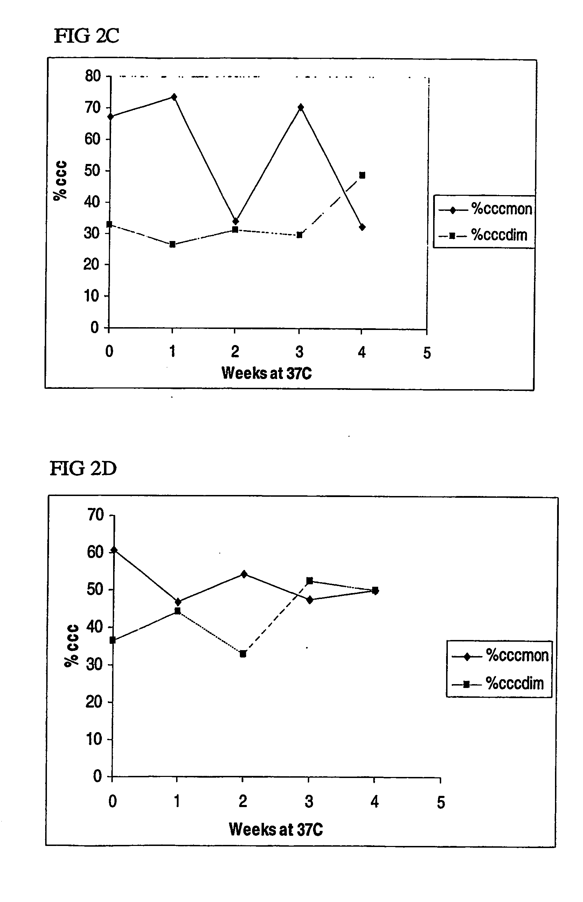

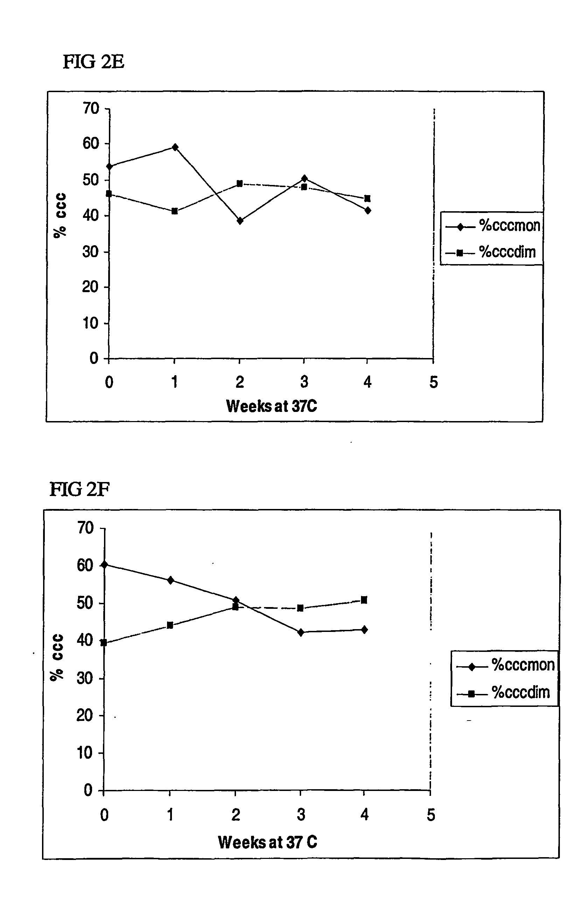

**FIG 2** shows a graphical plot of the percentage of supercoiled plasmid, (%ccc), both monomeric, (%cccmon), and dimeric, (%cccdim), plasmid forms; after coating and lyophilization onto sowing needles and storage at 37°C. The plasmid formulations used contain varying amounts of sugars: FIG 2A: 5% Sucrose, FIG 2B: 10% Sucrose, FIG 2C: 17.5% Sucrose, FIG 2D: 40% Sucrose, FIG 2E: 40% Trehalose, FIG 2F: 40% Glucose.

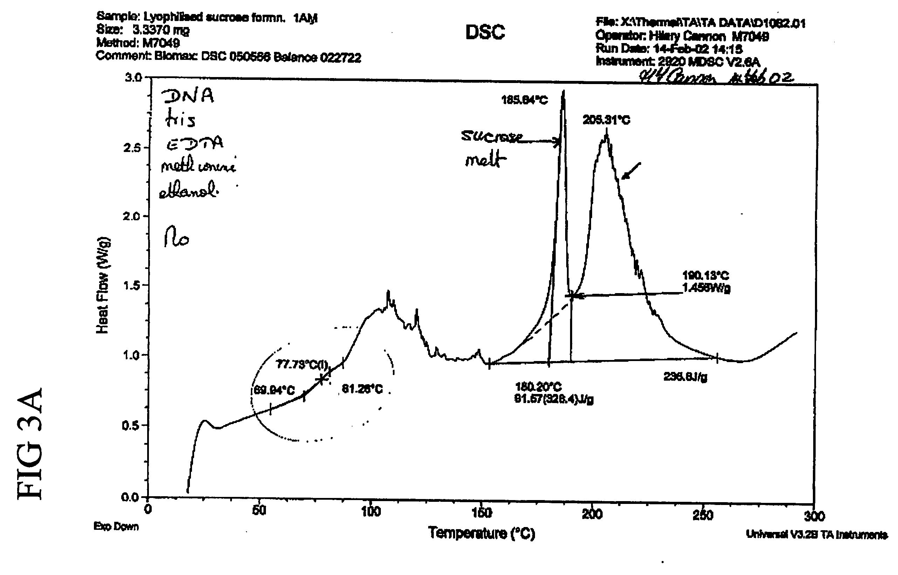

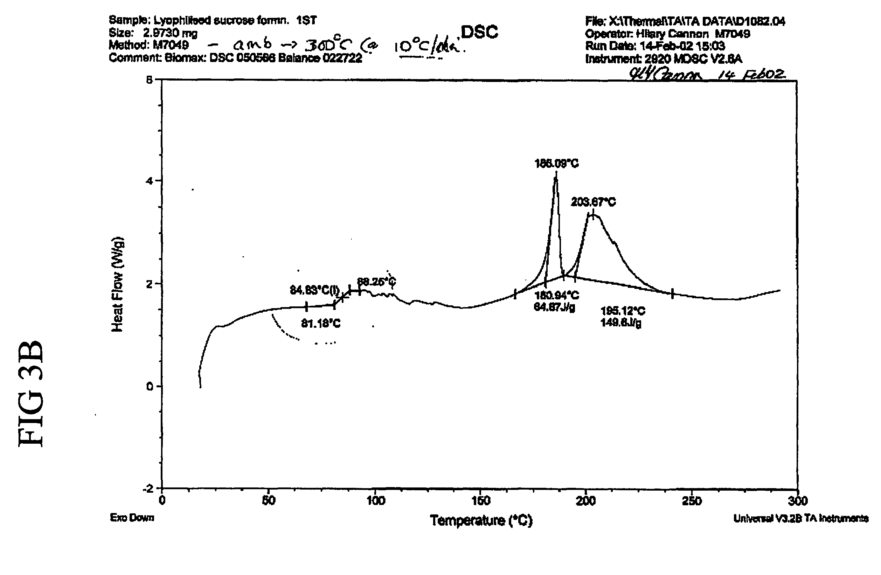

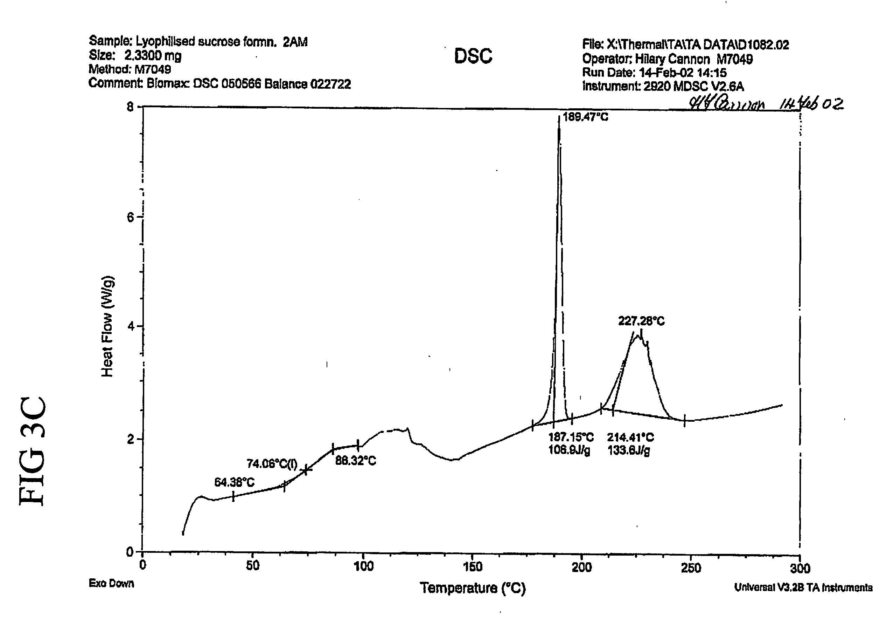

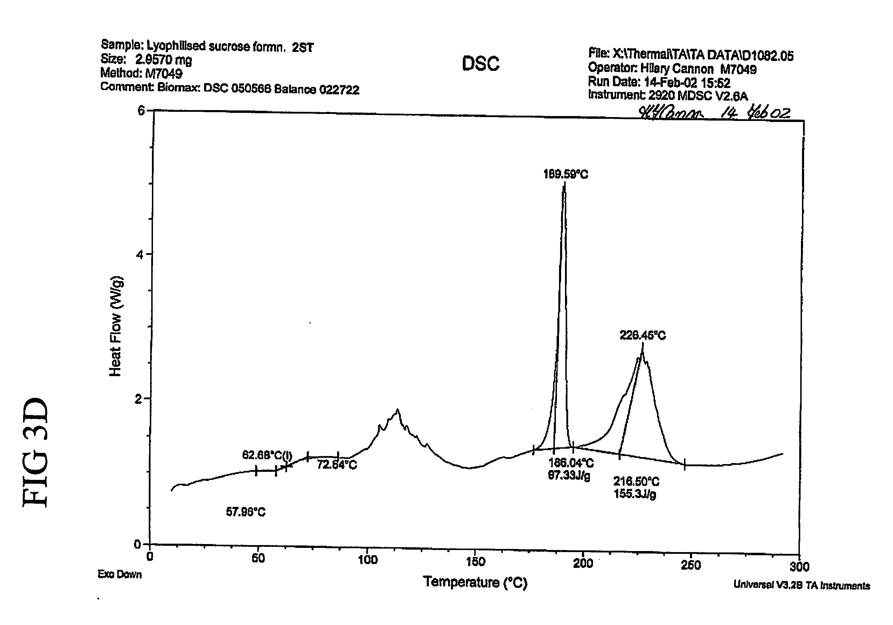

**FIG 3** shows differential scanning calorimetry, (DSC), data, for plasmid DNA, (10mg/ml), formulations in 40% sucrose. Fig 3A & B: formulations also contain: 100mM TrisHCl pH8.0, 1mM EDTA, 10mM methionine and 2.9% ethanol; Fig 3A & C represent a 24 hour lyophilization cycle; Fig 3B & D represent a 1 hour lyophilization cycle.

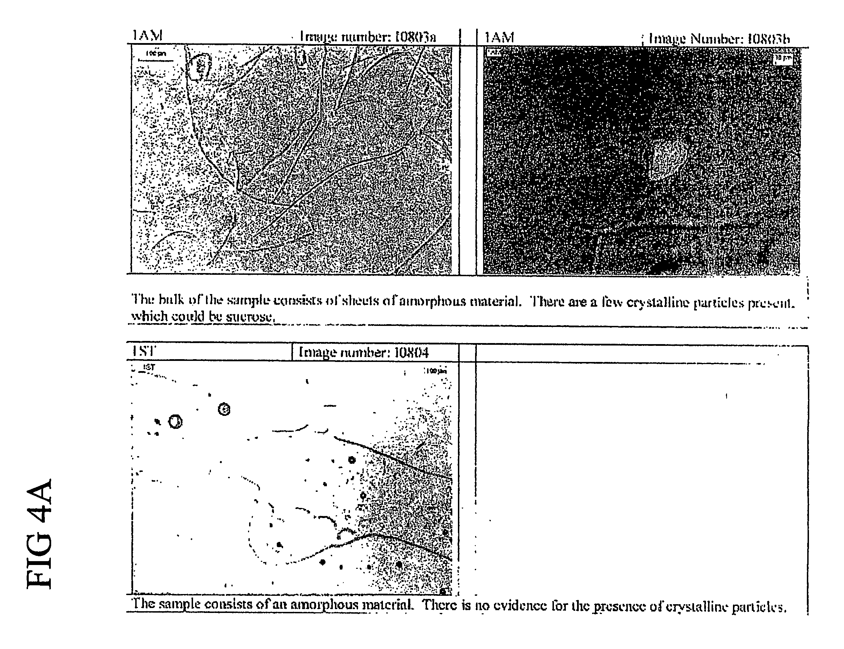

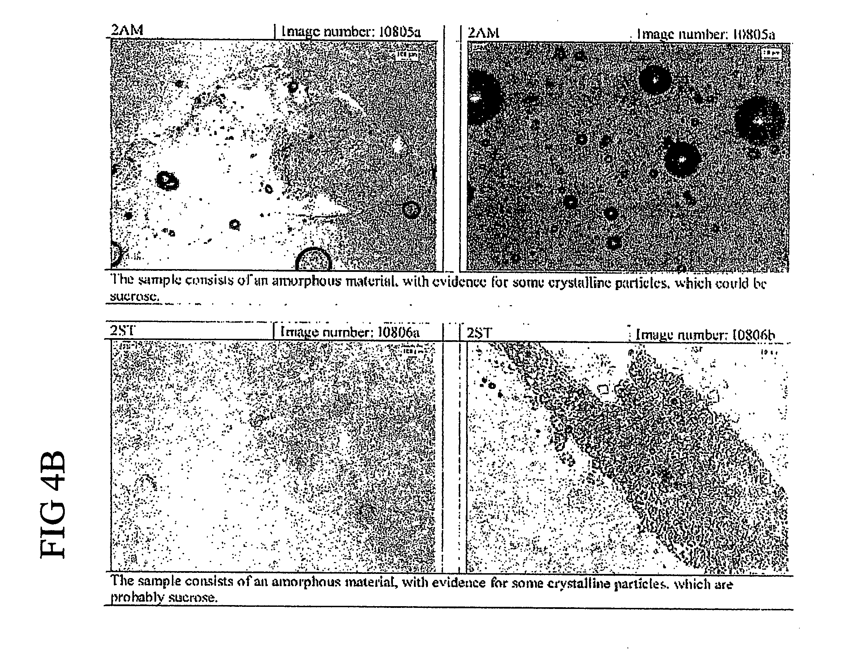

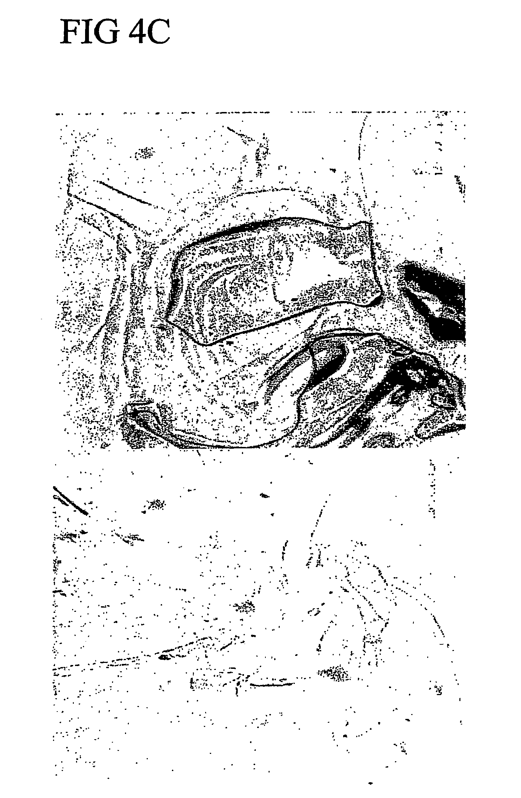

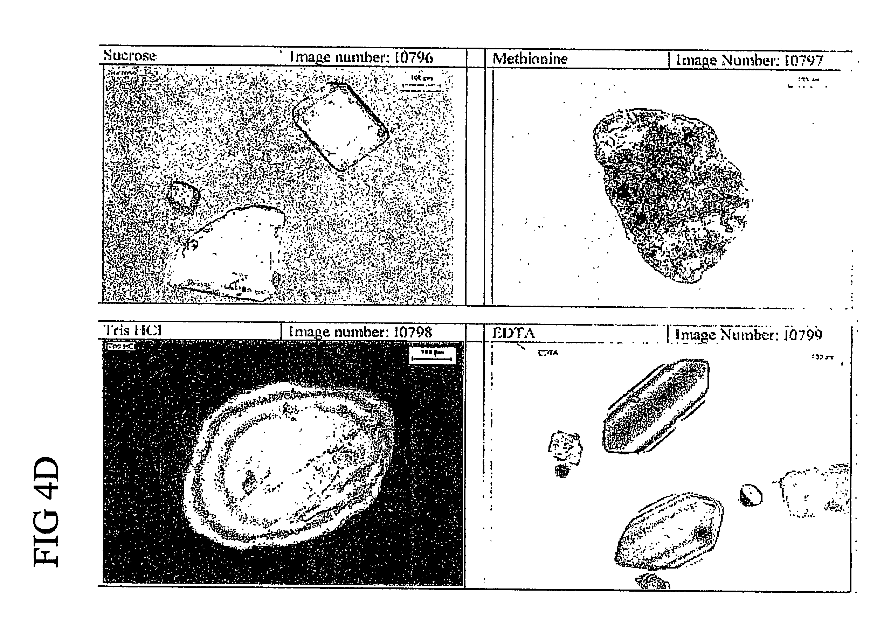

**FIG 4** shows polarized light microscopy data, for plasmid DNA, (10mg/ml), formulations in 40% sucrose. Fig 4A: formulations also contain: 100mM TrisHCl pH8.0, 1mM EDTA, 10mM methionine and 2.9% ethanol, Fig4C: only contains 40% sucrose and Fig 4D: shows crystals of the excipients described in the formulation shown in Fig 4A. 1AM, 2AM & 3AM represent a 24 hour lyophilization cycle, whereas 1ST, 2ST & 3ST represent a 1 hour lyophilization cycle.

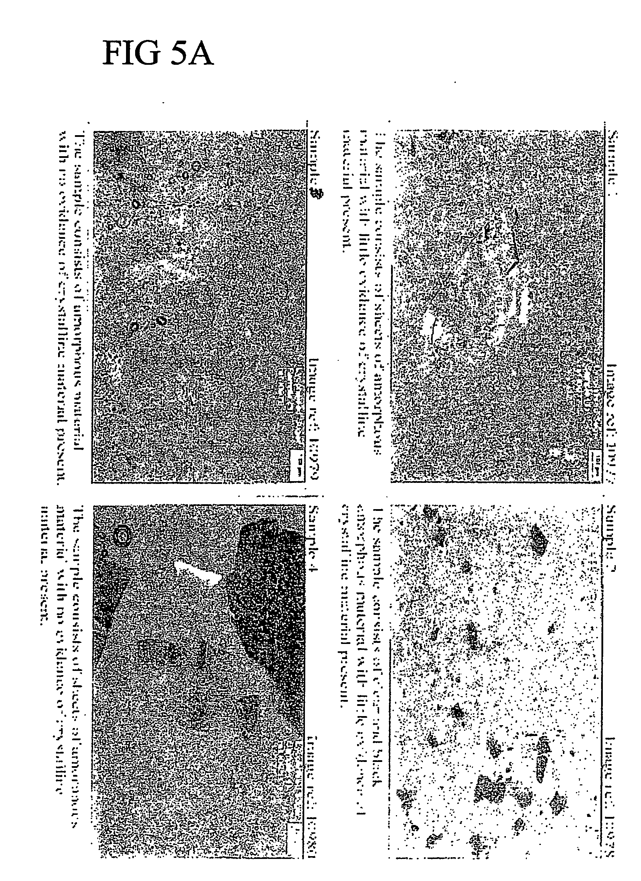

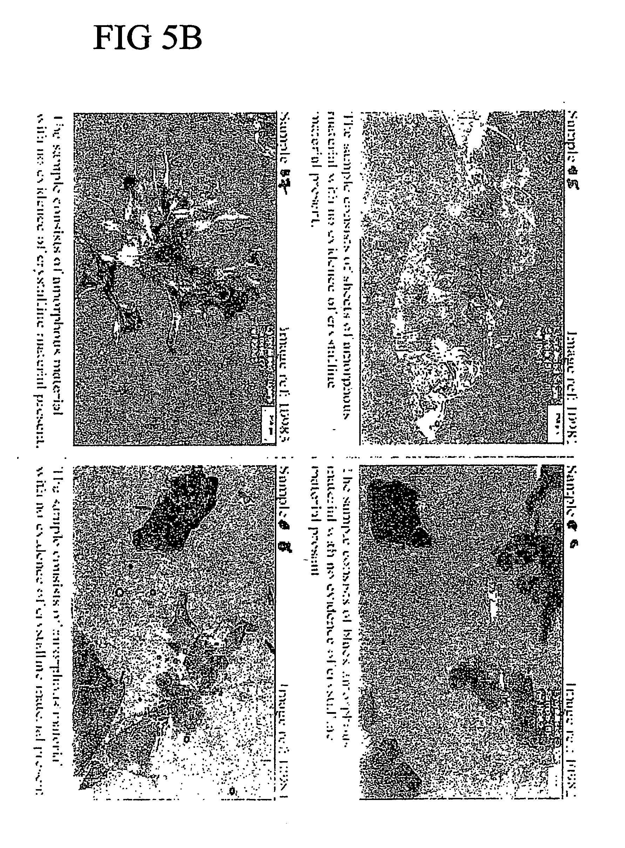

**FIG 5** shows polarized light microscopy data, for lyophilisized plasmid DNA, (10mg/ml), formulations in sugars and polyols, which also contain: 100mM TrisHCl pH8.0, 1mM EDTA, 10mM methionine and 2.9% ethanol. Fig 5A, sample 1: 40% w/v ficoll, sample 2: 20% w/v dextran, sample 3: 40% w/v sucrose, sample 4: 20% w/v maltotriose. Fig 5B, sample 5: 20% w/v lactose, sample 6: 30% w/v maltose, sample 7: 40% w/v glucose, sample 8: 40% w/v trehalose.

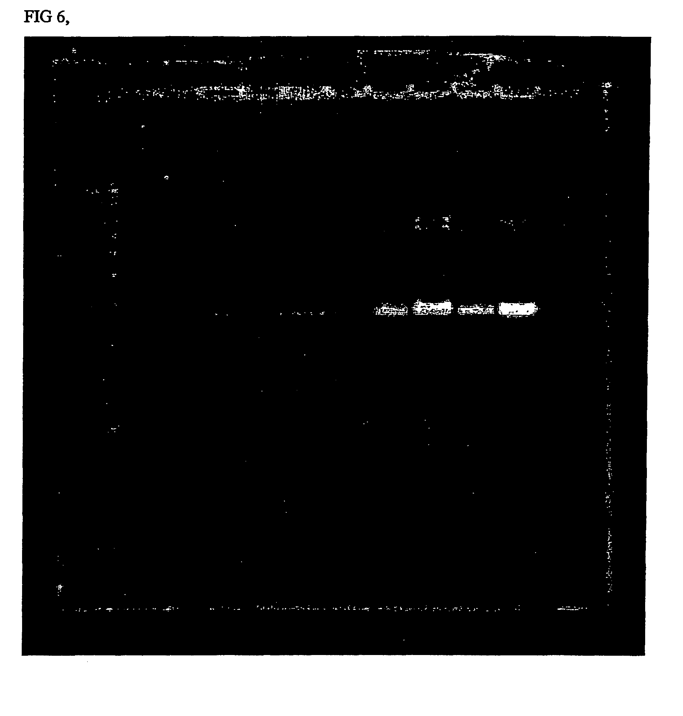

**FIG 6** shows the stability of supercoiled DNA plasmid coated onto gold beads and stored for 1 week at 25°C.

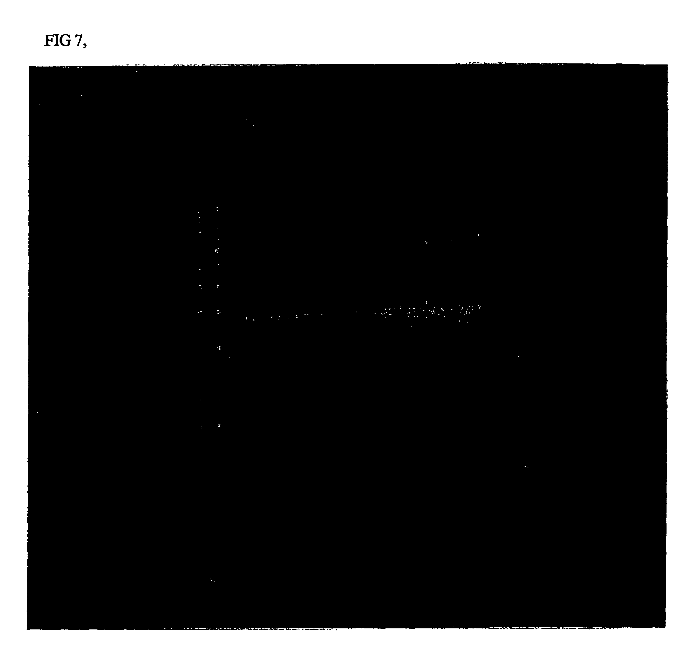

**FIG 7** shows the stability of supercoiled DNA plasmid coated onto gold beads and stored for 3 weeks at 25°C.

The present invention provides a novel DNA pharmaceutical agent dosage form, having a dense core element which is coated with a solid reservoir medium containing the DNA pharmaceutical agent.

DNA vaccine dosage forms are a preferred aspect of the present invention. In such applications the agent to be delivered is a polynucleotide that encodes an antigen or antigens derivable from a pathogen such as micro-organisms or viruses, or may be a self antigen in the case of a cancer vaccine or other self antigen.

The DNA component of the present invention may be linear or open circular or supercoiled plasmid DNA, but may in a related form of the present invention the DNA may be in the form of a live attenuated bacterial or viral vector.

Certain embodiments of the device described herein also have the significant advantage of being stored at room temperature thus reducing logistic costs and releasing valuable refrigerator space for other products.

The solid amorphous reservoir medium is preferably a polyol that fulfils the function required for the present invention. The reservoir must be capable of adhering to the microbead to a sufficient extent that the reservoir remains physically stable and attached during prolonged storage, and also remains substantially intact during the administration procedure when the coated microbead is projected through the *stratum corneum.* The reservoir must also be capable of holding or containing a suspension or solution of agent to be delivered in any dry or partially dry form, which is released into the skin during biodegradation of the reservoir medium.

Biodegradation of the medium in the sense of the present invention means that the reservoir medium changes state, such that changes from its non-releasing to its releasing states whereby the agent enters into the skin. The release of the active agent may involve one or more physical and/or chemical processes such as hydration, diffusion, phase transition, crystallisation, dissolution, enzymatic reaction and/or chemical reaction. Depending on the choice of reservoir medium, biodegradation can be induced by one or more of the following: water, body fluids, humidity, body temperature, enzymes, catalysts and/or reactants. The change of the reservoir medium may therefore be induced by hydration, and warming associated with the higher humidity and temperature of the skin. The reservoir medium may then degrade by dissolution and/or swelling and/or change phase (crystalline or amorphous), thereby disintegrating or merely increase the permeation of the medium.

Preferably the medium dissolves, and is metabolised or expelled or excreted from the body, but the reservoir may alternatively remain attached to microbead which may be expelled from the body by several mechanisms including sloughing off of dead skin cells during normal skin replacement. Release of the agent by dissolution of the reservoir medium is preferred.

Preferably the solid reservoir medium is a polyol (such as those described in WO 96/03978). Suitable polyol reservoir media include carbohydrates (such as sugars), polysaccharides, substituted polyols such as hydrophobically derivatised carbohydrates, amino acids, biodegradable polymers or co-polymers such as poly(hydroxy acid)s, polyahhydrides, poly(ortho)esters, polyurethanes, poly(butyric acid)s, poly(valeric acid)s, and poly(lactide-co-caprolactone)s, or polylactide co-glycolide.

The solid reservoir may be in an amorphous or crystalline state and may also be partially amorphous and partially crystalline. Most preferably, however, all or substantially all of the reservoir is in an amorphous state. More preferably still is that the amorphous reservoir is in the form of a glass (US 5,098,893). Most preferably the reservoir is a sugar glass. A glass reservoir may have any glass transition temperature, but preferably it has a glass transition temperature that both stabilises the pharmaceutical agent during storage and also facilitates rapid release of the agent after insertion of the reservoir into the skin. Accordingly, the glass transition temperature is greater than 30-40°C, but most preferably is around body temperature (such as, but not limited to 40-50°C).

Particularly preferred reservoir media are those that stabilise the agent to be delivered over the period of storage. For example, antigen or agent dissolved or dispersed in a polyol glass or simply dried in a polyol are storage stable over prolonged periods of time (US 5,098,893, US 6,071,428; WO 98/16205; WO 96/05809; WO 96/03978; US 4,891,319; US 5,621,094; WO 96/33744). Such polyols form the preferred set of reservoir media.

Preferred polyols include sugars, including mono, di, tri, or oligo saccharides and their corresponding sugar alcohols. Suitable sugars for use in the present invention are well known in the art and include, trehalose, sucrose, lactose, fructose, galactose, glucose, mannose, maltulose, iso-maltulose and lactulose, maltose, or dextrose and sugar alcohols of the aforementioned such as mannitol, lactitol and maltitol. Sucrose, Glucose, Lactose, Raffinose and Trehalose are preferred.

The reservoir mediums of the present invention may preferably further contain a stabilising agent that inhibits the degradative effects of free radicals. Preferred stabilising agents include stabilising metal ion chelating agents, such preferred metal ion chelating agents include inositol hexaphosphate, tripolyphosphate, succinic and malic acid, ethylenediamine tetraacetic acid (EDTA), tromethamine (TRIS), Desferal, diethylenetriaminepentaacetic acid (DTPA) and ethylenediamindihydroxyphenylacetic acid (EDDHA). Other preferred stabilising agents are non-reducing free radical scavengers, and preferably such as agents are ethanol, methionine or glutathione. Other suitable chelators and scavengers (and those which are not suitable) maybe readily identified by the man skilled in the art by routine experimentation (as described in WO 97/40839).

The preferred solid reservoir media in the devices of the present invention contain a metal ion chelating agent or a non-reducing free radical scavenger. Most preferably the solid reservoir media in the devices of the present invention contain both a metal ion chelating agent and a non-reducing free radical scavenger.

The amounts of the stabilising agents may be determined by the man skilled in the art, but generally are in the range of 0.1- 10mM for the metal ion chelators, Ethanol is present in an amount up to about 5% (v/v), methionine is present at about 0.1 to 100mM and Glutathione is present at about 0.1 to 10% (v/v).

Preferred combinations of stabilising agents are (a) Phosphate buffered ethanol solution in combination with methionine or EDTA, (b) Tris buffered EDTA in combination with methionine or ethanol (or combinations of methionine and ethanol).

Particularly preferred formulations which may be combined with DNA and coated onto the dense core elements to form solid dosage forms of the present invention contain polyols (preferably sucrose or trehalose) dissolved in demetalated water or Phosphate or Tris based buffers and further comprising either:
A. 10mM methionine and 2.9% ethanol, or
B. 3.7% ethanol and 1mM EDTA, or
C. 100mM Tris, 1mM EDTA and 10mM methionine and 2.9% ethanol, or
D. 100mM Tris, 1mM EDTA and 10mM methionine, or
E. 100mM Tris, 1mM EDTA and 2.9% ethanol.

In the preferred methods of manufacture of the present invention the DNA is stored and handled in these stabilising agents prior to final formulation with the sugar.

In addition to these stabilising agents, further steps may be taken to enhance the stability of the DNA in the solid vaccines. For example, the formulations may be made using solutions which themselves were demetalated before use (for example by using commercially available demetalating resin such as Chelex 100 from Biorad) and/or the formulation may be finalised in a high pH (such as pH 8-10).

Preferably the DNA is in the form of a supercoiled plasmid. One major advantage of the present invention for these formulations is the fact that the DNA is stabilised so that upon release, it largely remains in its supercoiled form, and preferably in its monomeric supercoiled form.

Plasmid DNA stability can be defined in a number of ways and can be a relative phenomenon determined by the conditions of storage such as pH, humidity and temperature. For storage in the presence of iron ions on the coated reservoir, preferably >50% of plasmid remains supercoiled, (ccc, covalently closed circular), upon storage for 3 months at 4°C. More preferably, under the storage conditions described, >60% of plasmid remains ccc and more preferably, under these storage conditions, >90% of plasmid remains ccc for 3 months at 4°C. For coating on to non - metal ion based needles or microneedles, the stability of plasmid DNA would be preferably >60% and more preferably 80% and most preferably >90% ccc after 3 months storage at 4°C. More preferably, under these storage conditions, >90% of plasmid remains ccc for 1 year at 4°C, and more preferably >90% of plasmid remains ccc for 2 years at 4°C. Most preferably the above DNA stability is achieved under these conditions over the same time periods at 25°C.

The DNA within a the solid reservoir medium (for ease of measurement, when coated onto sewing needles) is preferably stabilised in its supercoiled (ccc) form during accelerated stability studies, and most preferably the DNA is stabilised in its monomeric ccc form. An example of an acellarated stability study is where dry coated needles are maintained at 37°C for 4 weeks followed by analysis of the DNA structure over time. In this type of study, preferably greater than 50% of the DNA remains in its ccc form, more preferably greater than 60% remains in its ccc form, more preferably greater than 70% remains in its ccc form, more preferably greater than 80% remains in its ccc form and most preferably greater than 90% remains in its ccc form. Under these conditions, and preferred levels of ccc, it is also preferred that the ratio of monomeric:dimeric ccc DNA is about 1 (such as within the range of 0.8-1.2, or more preferably within the range of 0.9-1.1 and most preferably within the range of 0.95-1.5), or greater than 1.

Studies to determine plasmid stability are well known to those skilled in the art and are described in (Evans *et al., Supra;* WO 97/40839). These include techniques to measure and quantify the percentage of supercoiled, ccc, plasmid DNA either by agarose gel electrophoresis, anion exchange HPLC, (Ferreira, G. *et al.,* 1999, *Pharm. Pharmacol. Commun.,* 5, pp57-59), or capillary gel electrophoresis, (Schmidt *et al., 1999, Anal. Biochem.,* 274, 235-240). The ratio of monomeric:dimeric ccc can be measured by image intensity analysis after agarose gel electrophoresis (in the absence of any intercalating agents) and EtBr staining, using commercially available software such as Labworks 4.0 running on a UVP Bioimaging system.

In the context of the present invention the solid reservoir medium coats the core elements in a manner that the resultant formulation is suitable for administration by ballistic delivery devices. Accordingly each core element may be fully or partially covered by the reservoir, or a plurality of elements may be trapped within a matrix of solid reservoir. In a related method of producing the dosage forms of the present invention, a large quantity of reservoir encompassing a large number of core elements may be ground into smaller particles which are suitable for administration by ballistic delivery devices.

Other suitable excipients which may be included in the formulation include buffers, amino acids, phase change inhibitors ('crystal poisoners') which may be added to prevent phase change of the coating during procesing or storage or inhibitors to prevent deleterious chemical reactions during processing or storage such Maillard reaction inhibitors like amino acids.

The solid dosage forms of the present invention are used in ballistic transfection of skin cells using devices that entrain the DNA coated particles in a gas flow. The particles pass through the stratum corneum and enter into a cell where the DNA is released and expressed by the host cell. Alternatively, the particle enters the extracellular space and releases the DNA therein. Accordingly, the core elements that are suitable for use in the present invention are those that are suitable for this purpose. The core elements impart upon the final dosage form sufficient strength and momentum to pierce the stratum corneum in any given ballistic delivery device. It is preferred that the core elements have sufficient density to impart sufficient momentum to the DNA coated particles, suitable dense cores have been found to be gold or tungsten microbeads. The size of the core elements is preferably that which imparts sufficient mass to give the required momentum to the DNA coated particles, whilst not being too large such that the skin cells suffer too much damage. Suitable core element particle sizes are those that when coated form particles of a mean diameter in the range of 0.5 to 100 µm, preferably between 1 to 50 µm, more preferably between 1 to 10µm, and most preferably around 2 µm in diameter.

In general the core elements are roughly spherical, although non-regular forms may be used. Most preferably the core elements are gold or tungsten microbeads.

The present invention claims that an amorphous sugar when present with metal particles and DNA will impart long-term stability to the formulation. Other excipients like surfactants and buffers may be included in the formulation.

Examples of methods for the preparation of such amorphous sugar containing formulations include:

### 1. Freeze-drying

Mix the solution containing sugar, DNA, gold particles and fill into glass vials. These vials are partially stoppered and loaded into a lyophilizer. The shelf temperature is then reduced to -45C leading to the product in the vials being frozen. After allowing all the vials to freeze, the condensor is chilled to sub -60C temperature. Primary drying is then carried out by raising the shelf temperature to approximately -30C while applying a vacuum of approximately 100 mT. During primary drying the water from the ice crystals that are formed is sublimated. After the primary drying is complete, the shelf temperature is raised to above ambient temperature and maximum vacuum is applied. The secondary drying removes any tightly bound water and dries the powder to achieve long term stability.

### 2. Spray-drying

Spray drying is a dehydration process that utilizes heat from a hot gas stream (usually air) to evaporate dispersed droplets created by atomization of a continuous liquid feed. Resulting powder products dry within a few seconds into fine particles. The feasibility of spray drying for generating therapeutic protein powders has been amply demonstrated ((Broadhead, J., Rouan, S.K.E., Hau, I, and Rhodes, C.T. 1994. J. Pharm. Pharmacol. 46: 458-467.; Mumenthaler, M., Hsu, C.C., and Pearlman, R. 1994. Pharm. Res. 11: 12-20)). In such an application to our formulation mixtures, the formulated DNA, gold particles and sugar solution will be fed into a spray dryer with a typical inlet temperature in the range of 50 to 150C typically at a flow rate between 0.1 and 10 mL/min. The resulting powder is dry and is collected from the collection chamber.

### 3. Spray freeze-drying

Spray freeze-drying is a process in which the solution containing the DNA, gold particles and sugars is sprayed onto trays containing dry ice or liquid nitrogen. This results in the instantaneous freezing of the droplets. The trays are then loaded into a lyophilizer and the particles are then freeze-dried according to the process described above.

Using these techniques each solid DNA delivery dose may be loaded with relatively high amounts of DNA. The formulations resulting from the above techniques may be used directly or after milling and sieving into reservoir medium coated dense core beads.

Preferably the vaccine formulations of the present invention contain DNA that encodes an antigen or antigenic composition capable of eliciting an immune response against a human pathogen, which antigen or antigenic composition is derived from HIV-1, (such as tat, nef, gp120 or gp160), human herpes viruses, such as gD or derivatives thereof or Immediate Early protein such as ICP27 from HSV1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B*. *parapertussis and B. bronchiseptica; Mycobacterium spp., including M. tuberculosis* (for example ESAT6, Antigen 85A, -B or -C), *M. bovis, M leprae, M. avium, M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E. coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae, S. flexnerii; Yersinia spp,* including *Y.* enterocolitica (for example a Yop protein), *Y. pestis, Y. pseudotuberculosis; Campylobacter spp,* including *C*. *jejuni* (for example toxins, adhesins and invasins) and *C*. *coli; Salmonella spp,* including *S*. *typhi, S. paratyphi, S. choleraesuis, S. enteritidis; Listeria spp.,* including *L. monocytogenes; Helicobacter spp,* including *H*. pylori (for example urease, catalase, vacuolating toxin); *Pseudomonas spp,* including *P. aeruginosa; Staphylococcus spp.,* including *S*. *aureus, S. epidermidis; Enterococcus spp., including E. faecalis, E. faecium; Clostridium spp.,* including *C*. tetani (for example tetanus toxin and derivative thereof), *C*. *botulinum* (for example botulinum toxin and derivative thereof), *C*. *difficile* (for example clostridium toxins A or B and derivatives thereof); *Bacillus spp.,* including *B. anthracis* (for example botulinum toxin and derivatives thereof); *Corynebacterium spp.,* including *C*. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA., OspC, DbpA, DbpB), *B. andersonii* (for example OspA, OspC, DbpA, DbpB), *B. hermsii; Ehrlichia spp.,* including *E*. *equi* and the agent of the Human Granulocytic Ehrlichiosis; *Rickettsia spp,* including *R. rickettsii; Chlamydia spp., including C. trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including E. *histolytica; Babesia spp.,* including *B. microti; Trypanosoma spp.,* including *T. cruzi; Giardia spp.,* including *G. lamblia; Leshmania spp.,* including *L. major; Pneumocystis spp.,* including *P*. *carinii; Trichomonas spp.,* including *T. vaginalis; Schisostoma spp.,* including *S*. *mansoni,* or derived from yeast such as *Candida spp.,* including *C*. *albicans; Cryptococcus spp.,* including *C*. *neoformans.* Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464).

In another embodiment of the present invention the DNA dosage form contains a DNA vaccine in combination with a non-DNA antigen such as a protein or polysaccharide antigen derived from a pathogen.

One of the advantages of the present invention is the ability to co-formulate the DNA agent together with additional active agents, an ability that has been limited with other solid DNA pharmaceutical agents. For example, the DNA vaccine may further comprise an agent to enhance uptake of the DNA into the cell, an adjuvant or other immunostimulant to improve and/or direct the immune response, and may also further comprise pharmaceutically acceptable excipient(s).

For example, the solid pharmaceutical reservoir medium may preferably contain a DNA condensing agent for example spermidine or PEI (polyethyleneimine). Other excipients which may be included in the formulation include buffers, amino acids, phase change inhibitors ('crystal poisoners') which may be added to prevent phase change of the coating during processing or storage or inhibitors to prevent deleterious chemical reactions during processing or storage such Maillard reaction inhibitors like amino acids.

A preferred additional agent to the co-entrapped within the reservoir medium with the DNA is a DNAase inhibitor. One example of a DNAase inhibitor which is preferred is aurinticarboxylic acid (ATA, Glasspool-Malone, J. *et al.,* (2000), Molecular Therapy 2: 140-146).

Vaccines of the present invention, may advantageously also include an immunologically effective adjuvant in solid solution together with the DNA. Alternatively the adjuvant may be associated with separate microbeads to the DNA coated microbead. Suitable adjuvants for vaccines of the present invention comprise those adjuvants that are capable of enhancing the antibody responses against the immunogen. Suitable immunostimulatory agents include, but this list is by no means exhaustive and does not preclude other agents: synthetic imidazoquinolines such as imiquimod [S-26308, R-837], (Dockrell and Kinghorn, 2001, Journal of Antimicrobial Chemotherapy, 48, 751-755; Harrison, et al. 'Reduction of recurrent HSV disease using imiquimod alone or combined with a glycoprotein vaccine', Vaccine 19: 1820-1826, (2001)); and resiquimod [S-28463, R-848] (Vasilakos, et al.' Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. ' Therapeutic potentiation of the immune system by costimulatory Schiff-base-forming drugs', Nature 377: 71-75 (1995)), cytokine, chemokine and co-stimulatory molecules as either protein or peptide, this would include pro-inflammatory cytokines such as GM-CSF, IL-1 alpha, IL-1 beta, TGF-alpha and TGF-beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, , other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha- tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll-like receptors to produce Th1-inducing cytokines, such as synthetic Mycobacterial lipoproteins, Mycobacterial protein p19, peptidoglycan, teichoic acid and lipid A.

Certain preferred adjuvants for eliciting a predominantly Th1-type response include, for example, a Lipid A derivative such as monophosphoryl lipid A, or preferably 3-de-O-acylated monophosphoryl lipid A. MPL^{®} adjuvants are available from Corixa Corporation (Seattle, WA ; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., *Science 273*:352*,* 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

In this aspect of the present invention the preferred immunostimulatory agent or adjuvant is immiquimod or other related molecules (such as resiquimod) as described in PCT patent application publication number WO 94/17043 (the contents of which are incorporated herein by reference).

In an embodiment of the invention, a polynucleotide is administered/delivered as "naked" DNA, for example as described in Ulmer et al., *Science 259:*1745-1749, 1993 and reviewed by Cohen, *Science 259*:1691-1692, 1993. The uptake of naked DNA may be increased by coating the DNA onto small microbeads beads, such as gold, or biodegradable beads, which are efficiently transported into the cells; or by using other well known transfection facilitating agents, such as Calcium Phosphate or DEAE dextran.

The amount of expressible DNA in each vaccine administration is selected as an amount which induces an immunoprotective response without significant adverse side effects in typical vaccinees. Such amount will vary depending upon which specific DNA construct is employed, however, it is expected that each dose will generally comprise 1-1000 µg of DNA, preferably 1-500 µg, more preferably 1-100 µg, of which 1 to 50µg is the most preferable range. An optimal amount for a particular vaccine can be ascertained by standard studies involving observation of appropriate immune responses in subjects. Following an initial vaccination, subjects may receive one or several booster immunisations adequately spaced.

Also provided by the present invention are ballistic delivery devices loaded with the DNA dosage forms of the present invention.

The formulations of the present invention may be used for both prophylactic and therapeutic purposes. Accordingly, the present invention provides for a method of treating a mammal susceptible to or suffering from an infectious disease or cancer, or allergy, or autoimmune disease. In a further aspect of the present invention there is provided a vaccine as herein described for use in medicine. Vaccine preparation is generally described in New Trends and Developments in Vaccines, edited by Voller et al., University Park Press, Baltimore, Maryland, U.S.A. 1978.

The present invention is exemplified by, but not limited to, the following examples.

### Example 1, Demonstration of coating of microbeads with a reservoir medium comprising plasmid DNA.

### Plasmid preparation and formulations.

The plasmids used in this study are all shown in FIG 1. pEGFP-C1 is a GFP expression vector, (Clontech, Palo Alto, California, USA). pGL3CMV is a luciferase expression vector based upon pGL3 Basic, (Promega Corporation., Madison, Wisconsin, USA), where the CMV immediate early promoter drives luciferase expression.
pVAC1.ova is a chicken ovalbumin expression plasmid, constructed by ligating PCR amplified cDNA encoding chicken ovalbumin from pUGOVA, into the expression vector pVAC1 pVAC1 is a modification of the mammalian expression vector, pCI, (Promega), where the multiple cloning site, from EcoRI to Bst ZI, has been replaced by the EMCV IRES sequence flanked 5' by unique Nhe I, Rsr II and Xho I and 3' by unique Pac I, Asc I and Not I restriction enzyme sites, amplified from pGL3Basic, (Promega). Supercoiled plasmid DNA, (low endotoxin), was purified on a large scale, aproximately 100mg yield, to high purity using a combination of alkaline SDS lysis, ultrafiltration and anion exchange column chromatography.
Plasmids were resuspended in TE, (10mM TrisHCl, 1mM EDTA), pH 8.0 at 1ug / ul. And determined as >95% supercoiled upon analysis by agarose gel electrophoresis.

Plasmids were formulated in a variety of solutions, for coating needles, by a standard large-scale ethanol precipitation procedure. The precipitated DNA was resuspended directly into the aqueous formulation solutions at concentrations of 0.5 to 12 ug/ul, (See Chapter 1, Molecular Cloning: A Laboratory Manual, Sambrook, J. *et al.,* 2^{nd} Edition, 1989, CSH laboratory Press, Cold Spring Harbor, New York, USA).

### 1.2 Freeze-drying

Mix the solution containing sugar (between 1-40% sucrose or trehalose), DNA plasmid, gold particles and fill into glass vials. These vials are partially stoppered and loaded into a lyophilizer. The shelf temperature is then reduced to -45C leading to the product in the vials being frozen. After allowing all the vials to freeze, the condensor is chilled to sub -60C temperature. Primary drying is then carried out by raising the shelf temperature to approximately -30C while applying a vacuum of approximately 100 mT. During primary drying the water from the ice crystals that are formed is sublimated. After the primary drying is complete, the shelf temperature is raised to above ambient temperature and maximum vacuum is applied. The secondary drying removes any tightly bound water and dries the powder to achieve long term stability.

### 1.3 Spray-drying

Spray drying is a dehydration process that utilizes heat from a hot gas stream (usually air) to evaporate dispersed droplets created by atomization of a continuous liquid feed. Resulting powder products dry within a few seconds into fine particles. The feasibility of spray drying for generating therapeutic protein powders has been amply demonstrated ((Broadhead, J., Rouan, S.K.E., Hau, L, and Rhodes, C.T. 1994. J. Pharm. Pharmacol. 46: 458-467.; Mumenthaler, M., Hsu, C.C., and Pearlman, R. 1994. Pharm. Res. 11: 12-20)). In such an application to our formulation mixtures, the formulated DNA, gold particles and sugar solution will be fed into a spray dryer with a typical inlet temperature in the range of 50 to 150C typically at a flow rate between 0.1 and 10 mL/min. The resulting powder is dry and is collected from the collection chamber.

### 1.4. Spray freeze-drying

Spray freeze-drying is a process in which the solution containing the DNA, gold particles and sugars is sprayed onto trays containing dry ice or liquid nitrogen. This results in the instantaneous freezing of the droplets. The trays are then loaded into a lyophilizer and the particles are then freeze-dried according to the process described above.

The formulations resulting from the above techniques may be used directly or after milling in conventional ballistic delivery devices, and expression in target cells may be followed by observing luciferase expression. The samples are also stable as measured by maintenance of supercoiled structure.

### Example 2, Stability of plasmid DNA, in different sugar formulations, after coating, lyophilization and storage on needles, at 37°C.

A comparison between the plasmid DNA stability of a series of different DNA formulations was performed where either the amount of sucrose or the type of sugar used in the formulation was varied. All other excipients previously found to be optimal for DNA stability and release were present in all formulations, (ie. 100mM TrisHCl pH8.0, 1mM EDTA, 10mM methionine and 2.9% ethanol). The formulations were compared for their ability to stabilise supercoiled plasmid DNA, after coating and lyophilisation onto needles, upon storage for up to one month at 37°C, (accelerated DNA stability study). Plasmid DNA (pVAC1.OVA) was then eluted and recovered in the standard manner and subject to agarose gel electrophoresis, (100V, 100mA for 2 hours), in the absence of intercalating agents, (Sambrook, J. *et al., supra*). The integrity of the eluted plasmid DNA was then monitored after staining with ethidium bromide and visualisation under UV light. The percentage of supercoiled monomeric and dimeric plasmid forms and also any linear and open circular forms from these samples were measured as image intensity using the Labworks 4.0 image analysis software on the UVP Bioimaging System.

The data is displayed in FIG 2, as a graphical plot of the percentage of supercoiled plasmid, (%ccc), both monomeric, (%cccmon), and dimeric, (%cccdim), plasmid forms; after coating and lyophilization onto sowing needles and storage at 37°C. The plasmid formulations used contain varying amounts of sugars: FIG 2A: 5% sucrose, FIG 2B: 10% sucrose, FIG 2C: 17.5% sucrose, FIG 2D: 40% sucrose, FIG 2E: 40% trehalose, FIG 2F: 40% glucose.

The data suggest that all the formulations containing sugars maintain a high degree of plasmid stability, even after storage at 37°C for up to one month, greater than 80% and up to 98% of the plasmid remains in a supercoiled form. For formulations containing sugar levels of 40%, (w/v), the balance between the monomeric and dimeric plasmid forms remains relatively constant, with the preferred monomeric form predominating in sugar formulations varying from trehalose to sucrose to glucose, (FIGs 2D, 2E & 2F). For formulations containing lower concentrations of sucrose, the dimeric form tends to predominate over the monomer, especially upon prolonged storage at 37°C, (FIGs 2A, 2B & 2C). In general the data are consistant with the higher the level of sugar present in the formulation leading to greater stability of plasmid DNA.

### Example 3, Demonstration of amorphous glass formation after lyophilization of plasmid DNA, in sucrose formulations containing excipients.

### Analysis of lyophilised, plasmid DNA formulations by differential scanning calorimetry, (DSC).

Samples of lyophilised DNA / sucrose formulations were prepared containing plasmid DNA (pVAC1.OVA), (10mg/ml), in 40% sucrose and also lyophilised samples were prepared additionally containing 100mM TrisHCl pH8.0, 1mM EDTA, 10mM methionine and 2.9% ethanol. Samples were split and subject to either 1 hour or 24 hour lyophilization cycles. The samples were then subject to analysis by differential scanning calorimetry, (DSC), to determine the solid state form. This was performed on a TA Instruments DSC2920 machine over a temperature range from 25°C to 300°C, using nitrogen as the purge gas with a flow rate of 20ml / min. The sample pan type was pinhole aluminium and the sample weight was determined on the day of analysis on a Mettler M3 balance.

The data is displayed in FIG 3. All samples contain plasmid DNA, (10mg/ml), in 40% sucrose. Fig 3A & B: formulations also contain: 100mM TrisHCl pH8.0, 1mM EDTA, 10mM methionine and 2.9% ethanol; Fig 3A & C represent a 24 hour lyophilization cycle; Fig 3B & D represent a 1 hour lyophilization cycle. The data suggest that all the samples contain amorphous sucrose with sucrose glass transition temperatures being observed at about 78°C, (Fig 3A), 85°C, (Fig3B), 74°C, (Fig3C) and 63°C, (Fig 3D), which fit well with published values in the literature. The data suggests that both short and long lyophilisation cycles can generate an amorphous sucrose glass. Amorphous glass can form in the presence of high plasmid DNA concentrations and also in the presence of all the described excipients. However, as it was unclear whether or not some crystalline material was present in the samples or had been formed during the DSC analysis itself then further samples were analysed by the technique of polarised light microscopy to determine the amorphous / crystalline nature of the samples.

### Analysis of lyophilised, plasmid DNA formulations by polarized light microscopy.

The lyophilised plasmid DNA / sucrose, (± excipients), samples prepared for DSC analysis, described above, were subject to analysis by polarized light microscopy. Control samples were prepared of simply 40% sucrose, lyophilised for 1 hour and 24 hour cycles and crystalline samples of sucrose and the major solid excipients: methionine, Tris HCl and EDTA were also analysed. This was for comparison and to note the appearance of any crystalline material present in the formulations. The analysis was performed on a Zeiss STD16-444111 polarised light microscope with samples mounted in immersion oil and covered.

The data is shown in FIG 4 where all formulations contain plasmid DNA, (10mg/ml), in 40% sucrose. Fig 4A: formulations also contain: 100mM TrisHCl pH8.0, 1mM EDTA, 10mM methionine and 2.9% ethanol, Fig 4C: only contains 40% sucrose and Fig 4D: shows crystals of the major solid excipient. 1AM, 2AM & 3AM represent a 24 hour lyophilization cycle, whereas 1ST, 2ST & 3ST represent a 1 hour lyophilization cycle.

From Fig. 4C it is clear that both 1 hour and 24 hour lyophilisation cycles performed on 40% sucrose alone generate solely an amorphous glass as expected. From Fig 4B, the addition of plasmid DNA, (10mg/ml) to the 40% sucrose formulation, although it allows largely for the formation of an amorphous glass, does enable the partial formation of some crystalline sucrose (2AM and 2ST samples consist of amorphous material with some evidence for some crystal particles, which could be sucrose). However, from Fig 4A, the addition of the excipients to the DNA /sucrose formulation reduces the amount of crystalline particle formulation in samples lyophilised for 24 hours (1AM, the bulk of the sample consists of sheets of amorphous material. There are few crystalline particles present), and for short lyophilisation cycles of 1 hour, there is no evidence for the formation of crystalline particles, simply an amorphous glass. This suggests that the addition of the described excipients to plasmid DNA in sucrose helps not only to improve DNA release and stability from degradation but also to help preserve the amorphous glass state upon short cycles of lyophilisation.

### Example 4, Demonstration of amorphous glass formation after lyophilization of plasmid DNA, in different sugar l polyol formulations containing excipients.

To determine if the nature of the polyol / sugar present in the plasmid DNA formulation with excipients, as described above, affected the ability of such formulations to generate an amorphous glass upon lyophilisation, the polyol was varied. A number of similar formulations that differed only in the polyol present were generated, lyophilised and analysed by polarised light microscopy. This was performed in a similar manner to that described in example 3 except that on this occasion an Olympus BX51 polarized light microscope was used.

The data is shown in FIG 5 where all formulations contain lyophilisized plasmid DNA (pVAC1.OVA), (10mg/ml), and 100mM TrisHCl pH8.0, 1mM EDTA, 10mM methionine and 2.9% ethanol. Fig 5A, sample 1: 40% w/v ficoll, sample 2: 20% w/v dextran, sample 3: 40% w/v sucrose, sample 4: 20% w/v maltotriose. Fig 5B, sample 5: 20% w/v lactose, sample 6: 30% w/v maltose, sample 7: 40% w/v glucose, sample 8: 40% w/v trehalose. Note that all the samples described and all the formulations analysed formed an amorphous glass with little or no evidence of crystalline material being present. Note that the formulation described as sample 2, containing 20% w/v dextran, was subsequently shown to have precipitated the plasmid DNA out of solution, by agarose gel electrophoretic analysis, (data not shown), and would therefore not be a preferred formulation. This data demonstrates that plasmid DNA plus the described excipients can be maintained, when lyophilised, in an amorhous glass state by a variety of polyols / sugars described in the literature, (Hatley, R & Blair, J., (1999), Journal of Molecular Catalysis B: Enzymatic 7: 11-19.).

### Example 5, Lyophilisation of Sugar/Gold/DNA formulations

The aim of this study was to lyophilise three sugar based DNA formulations containing gold particles. The formulations were formed as shown below;
- Formulation 1 made up of 40% Sucrose, 100mM TrisHCl pH8.0, 10mM L-methionine and 2.9% ethanol
- Formulation 2 contained 10% Sucrose, 100mM TrisHCl pH8.0, 10mM L-methionine and 2.9% ethanol
- Formulation 3 was made up of 40% Trehalose, 100mM TrisHCl pH8.0, 10mM L-methionine and 2.9% ethanol

Supercoiled hepatitis B plasmid was formulated into each of the three formulations at a concentration of 1mg/mL. Gold particles were added to each of the formulations at a concentration of 0.5g per 10mL of formulation. The formulations were snap frozen by dropwise addition of each formulation in liquid nitrogen. The resulting frozen beads were transferred to 3mL freeze-drying vials. The vials were freeze dried in a DW8 Heto Holten freeze dryer using the cycle shown below;

| **Stage of cycle** | **Temperature (°C/Vacuum (hPa)** | **Time (hrs)** |
|---|---|---|
| Freezing stage | To -40°C | As quickly as possible |
| Hold | -40°C | 3 |
| Primary drying | -38°C/Vacuum (0.107hPa) | 8 |
| Secondary drying (ramp) | -38°C to 5°C/Vacuum (as low as possible) | 11 |
| Secondary drying (ramp) | 5°C to 10°C/Vacuum (as low as possible) | 2 |
| Secondary drying (hold) | 10°C/Vacuum (as low as possible) | 2 |

The freeze-dryed samples were stoppered under vacuum. The vacuum was released and the vials removed from the freeze dryer. An accelerated stability study (at 25°C) was set up using the freeze-dried DNA formulations. The stability study was monitored at weekly intervals using ethidium bromide stained agarose gel electrophoresis.

### Agarose gel electrophoresis

A 0.6% agarose gel electrophoresis was carried out on stability samples in order determine a change in DNA conformation during the study. Each lyophilised sample was reconstituted in distilled water and added to its respective well of the agarose gel using the following combination of sample, loading buffer and distilled water. 2µl of sample + 16µl of distilled water + 2µl of loading buffer

20µls of each sample was added to the respective well of the gel. The samples were electrophoresed overnight at 20 Volts. The electrphoresed gel was stained with ethidium bromide and viewed under UV. The stability samples were assayed following one and three weeks' storage at 25°C

### Results

The photographs of the gels containing the one and three week samples are shown in FIGs 6 and 7. FIG 6; shows the DNA profile of samples stored at 25°C for 1 week (lane 1 on left hand side): Lane 1 - 1 kilobase ladder; Lane 2 - Freeze-dried sample (Formulation 1) at 25°C following one week storage; Lane 3 - Freeze-dried sample (Formulation 1) at 5°C after one week storage - Control; Lane 4 - Freeze-dried sample (Formulation 2) at 25°C following one week storage, Lane 5 - Freeze-dried sample (Formulation 2) at 5°C following one week storage; Lane 6 - Freeze-dried sample (Formulation 3) at 25°C following one week storage; Lane 7 - Freeze-dried sample (Formulation 3) at 5°C following one week storage; Lane 8 - Pre freeze-dried liquid Formulation 1 at 5°C; Lane 9 - Pre freeze-dried liquid Formulation 2 at 5°C Lane 10 - Pre freeze-dried liquid Formulation 3 at 5°C; Lane 11 - Unformulated plasmid, GW700561X (batch A01B30); Lane 12 - Freeze-dried Negative control of Formulation 1 consisting of Formulation 1 diluent + gold beads without DNA plasmid

The results showed that
- No significant change in conformation was detected for any of the three freeze-dried formulations (at 25°C) when compared to the controls at 5°C. Most of the DNA was found to be supercoiled although relatively small amounts of opencircular and linear topoisoforms were also detected.
- The pre-freeze dried liquid formulations produced similar band profiles to the post freeze-dried samples. The higher amount of fluorescence detected in pre-freeze dried samples is probably due to the higher concentration of DNA in these.
- No significant difference in DNA profile detected between the formulation and unformulated plasmid (lane 11).
- As expected, no bands were seen in lane 12 - the negative control.

Figure 7 shows the DNA profile of samples stored at 25°C for 3 week With Lane 1 on the left hand side: Lane 1 - 1 kilobase ladder; Lane 2 - Freeze-dried sample (Formulation 1) at 25°C following three weeks storage; Lane 3 - Freeze-dried sample (Formulation 1) at 5°C after three week storage - Control; Lane 4 - Freeze-dried sample (Formulation 2) at 25°C following three weeks storage; Lane 5 - Freeze-dried sample (Formulation 2) at 5°C following three weeks storage; Lane 6 - Freeze-dried sample (Formulation 3) at 25°C following three weeks storage; Lane 7 - Freeze-dried sample (Formulation 3) at 5°C following three weeks storage; Lane 8 - Pre freeze-dried liquid Formulation 1 at 5°C; Lane 9 - Pre freeze-dried liquid Formulation 2 at 5°C; Lane 10 - Pre freeze-dried liquid Formulation 3 at 5°C; Lane 11 - Unformulated plasmid; Lane 12 - Freeze-dried Negative control of Formulation 1 consisting of Formulation 1 diluent + gold beads without DNA plasmid.

The results showed that
- No significant change in conformation was detected for any of the three freeze-dried formulations (at 25°C) when compared to the controls at 5°C. Most of the DNA was found to be supercoiled although relatively small amounts of opencircular and linear isoforms were also detected.
- The pre-freeze dried liquid formulations produced similar DNA profiles to the post freeze-dried samples. The higher amount of fluorescence detected in pre-freeze dried samples is probably due to the higher concentration of DNA in these.

### Conclusions

In this study we have demonstrated that it is possible to freeze dry sugar/gold based DNA formulations without a significant change in the DNA conformation. In addition, we have shown that the resulting lyophilised beads were stable at 25°C for a period of three weeks.

## Claims

1. A DNA pharmaceutical agent dosage form, having a dense core element coated with a solid reservoir medium containing the DNA pharmaceutical agent.

2. A DNA pharmaceutical agent dosage form as claimed in claim 1, further comprising a stabilising agent that inhibits the degradative effects of free radicals.

3. A DNA pharmaceutical agent dosage form as claimed in claim 2 wherein the stabilising agent is one or both of a metal ion chelator and a free radical scavenger.

4. A DNA pharmaceutical agent dosage form as claimed in claim 3 wherein the metal ion chelating agent is selected from the group consisting of inositol hexaphosphate, tripolyphosphate, succinic and malic acid, ethylenediamine tetraacetic acid (EDTA), tris (hydroxymethyl) amino methane (TRIS), Desferal, diethylenetriaminepentaacetic acid (DTPA) and ethylenediamindihydroxyphenylacetic acid (EDDHA).

5. A DNA pharmaceutical agent dosage form as claimed in claim 3 wherein the non-reducing free radical scavenger is selecting from the group consisting of ethanol, methionine or glutathione.

6. A DNA pharmaceutical agent dosage form as claimed in claim 3 wherein the stabilising agent that inhibits the degradative effects of free radicals, is (a) Phosphate buffered ethanol solution in combination with methionine or EDTA, or (b) Tris buffered EDTA in combination with methionine or ethanol (or combinations of methionine and ethanol).

7. A DNA pharmaceutical agent dosage form as claimed in any one of claims 1 to 6, wherein the solid reservoir medium is an amorphous polyol.

8. A DNA pharmaceutical agent dosage form as claimed in claim 7, wherein the polyol is a stabilising polyol.

9. A DNA pharmaceutical agent dosage form as claimed in any one of claims 1 to 8 wherein the solid biodegradable reservoir medium is a sugar.

10. A DNA pharmaceutical agent dosage form as claimed in claim 9 wherein the sugar is selected from lactose, glucose, sucrose, raffinose or trehalose.

11. A DNA pharmaceutical agent dosage form as claimed in any one of claims 1 to 10 wherein the solid reservoir medium is in the form of a glass.

12. A DNA pharmaceutical agent dosage form as claimed in claim 11, wherein the solid reservoir medium is in the form of a sugar glass.

13. A DNA pharmaceutical agent dosage form as claimed in any one of claims 1 to 12, wherein the DNA is supercoiled plasmid DNA

14. A DNA pharmaceutical agent dosage form as claimed in claim 13, wherein the supercoiled plasmid DNA is stabilised such that after storage at 37°C for 4 weeks greater than 50% of the DNA remains in its supercoiled form.

15. A DNA pharmaceutical agent dosage form as claimed in claim 13, wherein the DNA is stabilised such that when released the ratio of monomer:dimer supercoiled form is within the range of 0.8:1.2.

16. A DNA pharmaceutical agent dosage form as claimed in any one of claims 1 to 15, wherein the pharmaceutical agent is a vaccine.

17. A DNA pharmaceutical agent dosage form as claimed in any one of claims 1 to 16, wherein the solid reservoir medium further comprises a vaccine adjuvant, transfection facilitating agent, DNAase inhibitor or a crystal poisoner.

18. A DNA pharmaceutical agent dosage form as claimed in claim 17, wherein the adjuvant is selected from the group consisting of CpG, a synthetic imidazoquinolines, tucerasol, cytokines, MPL, QS21, QS7 and oil in water emulsions.

19. A DNA pharmaceutical agent dosage form, as claimed in claim 1 wherein the dense core elements are microbeads of a mean particle diameter of between 0.5 to 10 µm.

20. A DNA pharmaceutical agent dosage form as claimed in claim 19, wherein the dense core element is a gold or tungsten microbead.

21. A process for the preparation of a DNA pharmaceutical agent dosage form as claimed in claim 1, comprising making a solution of DNA pharmaceutical agent, reservoir medium, and stabilising agent that inhibits the degradative effects of free radicals in an solvent, followed by coating the at least one dense core element with said solution, and removing the solvent to form a solid reservoir medium containing the pharmaceutical agent and agent that inhibits the degradative effects of free radicals.

22. A process for the preparation of a DNA pharmaceutical agent dosage form as claimed in claim 21, wherein the reservoir medium is a sugar.

23. A process for the preparation of a DNA pharmaceutical agent dosage form as claimed in claim 22 wherein the concentration of sugar prior to drying onto the support member is in the range of 20-40% w/v.

24. A process for the preparation of a DNA pharmaceutical agent dosage form as claimed in claim 23, wherein the solvent is demetalated prior to the process.

## Patentansprüche

1. DNA-Pharmazeutikum-Arzneiform mit einem dichten Kernelement, das mit einem festen Reservoirmedium überzogen ist, das das DNA-Pharmazeutikum enthält.

2. DNA-Pharmazeutikum-Arzneiform gemäß Anspruch 1, die ferner ein Stabilisierungsmittel umfaßt, das die abbauenden Wirkungen von freien Radikalen inhibiert.

3. DNA-Pharmazeutikum-Arzneiform gemäß Anspruch 2, worin das Stabilisierungsmittel eines oder beide aus einem Metallionen-Komplexbildner und einem freien Radikalfänger ist.

4. DNA-Pharmazeutikum-Arzneiform gemäß Anspruch 3, worin der Metallionen-Komplexbildner aus der Gruppe ausgewählt ist, die aus Inosithexaphosphat, Tripolyphosphat, Bernsteinsäure und Äpfelsäure, Ethylendiamintetraessigsäure (EDTA), Tris(hydroxymethyl)aminomethan (TRIS), Desferal, Diethylentriaminpentaessigsäure (DTPA) und Ethylendiamindihydroxyphenylessigsäure (EDDHA) besteht.

5. DNA-Pharmazeutikum-Arzneiform gemäß Anspruch 3, worin der nicht-reduzierende freie Radikalfänger aus der Gruppe ausgewählt ist, die aus Ethanol, Methionin oder Glutathion besteht.

6. DNA-Pharmazeutikum-Arzneiform gemäß Anspruch 3, worin das Stabilisierungsmittel, das die abbauenden Wirkungen von freien Radikalen inhibiert, (a) phosphatgepufferte Ethanollösung in Kombination mit Methionin oder EDTA oder (b) Tris-gepuffertes EDTA in Kombination mit Methionin oder Ethanol (oder Kombinationen aus Methionin und Ethanol) ist.

7. DNA-Pharmazeutikum-Arzneiform gemäß einem der Ansprüche 1 bis 6, worin das feste Reservoirmedium ein amorphes Polyol ist.

8. DNA-Pharmazeutikum-Arzneiform gemäß Anspruch 7, worin das Polyol ein stabilisierendes Polyol ist.

9. DNA-Pharmazeutikum-Arzneiform gemäß einem der Ansprüche 1 bis 8, worin das feste biologisch abbaubare Reservoirmedium ein Zucker ist.

10. DNA-Pharmazeutikum-Arzneiform gemäß Anspruch 9, worin der Zucker aus Lactose, Glucose, Saccharose, Raffinose oder Trehalose ausgewählt ist.

11. DNA-Pharmazeutikum-Arzneiform gemäß einem der Ansprüche 1 bis 10, worin das feste Reservoirmedium in Form eines Glases ist.

12. DNA-Pharmazeutikum-Arzneiform gemäß Anspruch 11, worin das feste Reservoirmedium in Form eines Zuckerglases ist.

13. DNA-Pharmazeutikum-Arzneiform gemäß einem der Ansprüche 1 bis 12, worin die DNA Supercoiled-Plasmid-DNA ist.

14. DNA-Pharmazeutikum-Arzneiform gemäß Anspruch 13, worin die Supercoiled-Plasmid-DNA so stabilisiert ist, daß nach Lagerung bei 37°C für 4 Wochen mehr als 50 % der DNA in ihrer Supercoiled-Form verbleiben.

15. DNA-Pharmazeutikum-Arzneiform gemäß Anspruch 13, worin die DNA so stabilisiert ist, daß bei Freisetzung das Verhältnis von monomerer zu dimerer Supercoiled-Form im Bereich von 0,8:1,2 ist.

16. DNA-Pharmazeutikum-Arzneiform gemäß einem der Ansprüche 1 bis 15, worin das Pharmazeutikum ein Impfstoff ist.

17. DNA-Pharmazeutikum-Arzneiform gemäß einem der Ansprüche 1 bis 16, worin das feste Reservoirmedium ferner einen Hilfsstoff für einen Impfstoff, ein die Transfektion erleichterndes Mittel, DNAase-Inhibitor oder ein Kristallgift umfaßt.

18. DNA-Pharmazeutikum-Arzneiform gemäß Anspruch 17, worin der Hilfsstoff aus der Gruppe ausgewählt ist, die aus CpG, synthetischen Imidazochinolinen, Tucerasol, Cytokinen, MPL, QS21, QS7 und Öl-in-Wasser-Emulsionen besteht.

19. DNA-Pharmazeutikum-Arzneiform gemäß Anspruch 1, worin die dichten Kernelemente Mikroperlen mit einem mittleren Teilchendurchmesser von 0,5 bis 10 µm sind.

20. DNA-Pharmazeutikum-Arzneiform gemäß Anspruch 19, worin das dichte Kernelement eine Gold- oder Wolfram-Mikroperle ist.

21. Verfahren zur Herstellung einer DNA-Pharmazeutikum-Arzneiform gemäß Anspruch 1, umfassend das Herstellen einer Lösung aus DNA-Pharmazeutikum, Reservoirmedium und Stabilisierungsmittel, das die abbauenden Wirkungen von freien Radikalen inhibiert, in einem Lösungsmittel, gefolgt von Beschichten des wenigstens einen dichten Kernelements mit der Lösung und Entfernen des Lösungsmittels zur Bildung eines festen Reservoirmediums, das das Pharmazeutikum und Mittel, das die abbauenden Wirkungen von freien Radikalen inhibiert, enthält.

22. Verfahren zur Herstellung einer DNA-Pharmazeutikum-Arzneiform gemäß Anspruch 21, worin das Reservoirmedium ein Zucker ist.

23. Verfahren zur Herstellung einer DNA-Pharmazeutikum-Arzneiform gemäß Anspruch 22, worin die Konzentration des Zuckers vor der Trocknung auf dem Trägerelement im Bereich von 20-40 % G/V ist.

24. Verfahren zur Herstellung einer DNA-Pharmazeutikum-Arzneiform gemäß Anspruch 23, worin das Lösungsmittel vor dem Verfahren entmetallisiert wird.

## Revendications

1. Forme posologique d'un agent pharmaceutique à ADN, ayant un élément central dense revêtu d'un milieu réservoir solide contenant l'agent pharmaceutique à ADN.

2. Forme posologique d'un agent pharmaceutique à ADN selon la revendication 1, comprenant en outre, un agent de stabilisation, qui inhibe les effets de dégradation des radicaux libres.

3. Forme posologique d'un agent pharmaceutique à ADN selon la revendication 2, dans lequel l'agent de stabilisation est un ou deux d'un chélate d'ion métallique et d'un piège à radicaux libres.

4. Forme posologique d'un agent pharmaceutique à ADN selon la revendication 3, où l'agent de chélation d'ion métallique est choisi parmi le groupe consistant en l'hexaphosphate d'inositol, le tripolyphosphate, l'acide succinique et l'acide malique, l'acide éthylènediaminetétraacétique (EDTA), le tris(hydroxyméthyl) aminométhane (TRIS), le desféral, l'acide diéthylènetriaminepentaacétique (DTPA) et l'acide éthylènediaminedihydrophénylacétique (EDDHA).

5. Forme posologique d'un agent pharmaceutique à ADN selon la revendication 3, où le piège à radicaux libres non réducteur est choisi parmi le groupe consistant en l'éthanol, la méthionine ou la glutathione.

6. Forme posologique d'un agent pharmaceutique à ADN selon la revendication 3, où l'agent de stabilisation, qui inhibe les effets de dégradation des radicaux libres, est (a) une solution d'éthanol tamponnée phosphate en combinaison avec la méthionine ou l'EDTA, ou (b) l'EDTA tamponné Tris en combinaison avec la méthionine ou l'éthanol (ou en combinaison avec la méthionine et l'éthanol).

7. Forme posologique d'un agent pharmaceutique à ADN selon l'une quelconque des revendications 1 à 6, où le milieu réservoir solide est un polyol amorphe.

8. Forme posologique d'un agent pharmaceutique à ADN selon la revendication 7, où le polyol est un polyol stabilisant.

9. Forme posologique d'un agent pharmaceutique à ADN selon l'une quelconque des revendications 1 à 8, où le milieu réservoir solide biodégradable est un sucre.

10. Forme posologique d'un agent pharmaceutique à ADN selon la revendication 9, où le sucre est choisi parmi le lactose, le glucose, le saccharose, le raffinose ou le tréhalose.

11. Forme posologique d'un agent pharmaceutique à ADN selon l'une quelconque des revendications 1 à 10, où le milieu réservoir solide à la forme d'un verre.

12. Forme posologique d'un agent pharmaceutique à ADN selon la revendication 11, où le milieu réservoir solide à la forme d'un verre de sucre.

13. Forme posologique d'un agent pharmaceutique à ADN selon l'une quelconque des revendications 1 à 12, où l'ADN est un ADN de plasmide superenroulé.

14. Forme posologique d'un agent pharmaceutique à ADN selon la revendication 13, où l'ADN de plasmide superenroulé est stabilisé de sorte qu'après stockage à 37°C pendant 4 semaines, plus de 50% de l'ADN restent en sa forme superenroulée.

15. Forme posologique d'un agent pharmaceutique à ADN selon la revendication 13, où l'ADN est stabilisé de sorte que lorsqu'il est libéré, le rapport forme superenroulée monomère:dimère se situe dans la gamme de 0,8:1,2.

16. Forme posologique d'un agent pharmaceutique à ADN selon l'une quelconque des revendications 1 à 15, où l'agent pharmaceutique est un vaccin.

17. Forme posologique d'un agent pharmaceutique à ADN selon l'une quelconque des revendications 1 à 16, où le milieu réservoir solide comprend en outre, un adjuvant de vaccin, un agent facilitant la transfection, un inhibiteur d'ADNase ou un poison de cristal.

18. Forme posologique d'un agent pharmaceutique à ADN selon la revendication 17, où l'adjuvant est choisi parmi le groupe consistant en le CpG, une imidazoquinoléine synthétique, le tucérasol, des cytokines, le MPL, le QS21, le QS7 et des émulsions huile dans eau.

19. Forme posologique d'un agent pharmaceutique à ADN selon la revendication 1, où les éléments centraux denses sont des microbilles d'un diamètre moyen des particules allant de 0,5 à 10 µm.

20. Forme posologique d'un agent pharmaceutique à ADN selon la revendication 19, où l'élément central dense est une microbille d'or ou de tungstène.

21. Procédé de préparation d'une forme posologique d'un agent pharmaceutique à ADN selon la revendication 1, comprenant la préparation d'une solution d'agent pharmaceutique à ADN, du milieu réservoir et de l'agent de stabilisation, qui inhibe les effets de dégradation des radicaux libres dans un solvant, suivie du revêtement d'au moins un élément central dense avec ladite solution, et l'élimination du solvant pour former un milieu réservoir solide contenant l'agent pharmaceutique et l'agent qui inhibe les effets de dégradation des radicaux libres.

22. Procédé de préparation d'une forme posologique d'un agent pharmaceutique à ADN selon la revendication 21, où le milieu réservoir est un sucre.

23. Procédé de préparation d'une forme posologique d'un agent pharmaceutique à ADN selon la revendication 22, où la concentration du sucre avant le séchage sur l'élément support se situe dans la gamme allant de 20-40% p/v.

24. Procédé de préparation d'une forme posologique d'un agent pharmaceutique à ADN selon la revendication 23, où le solvant est démétallisé avant le processus.
